# EUROPEAN PATENT SPECIFICATION

(11) **EP 4 294 484 B1**
(45) Date of publication and mention of the grant of the patent: **26.11.2025**
(21) Application number: 22708225.2
(22) Date of filing: 11.02.2022
(51) Int. Cl.: A61M 11/00, A61M 11/02, A61M 11/06, A61M 15/00

(54) **APPARATUS FOR ADMINISTERING A PREDEFINED AMOUNT OF A COMPOUND**
VORRICHTUNG ZUR VERABREICHUNG EINER VORBESTIMMTEN MENGE EINER VERBINDUNG
APPAREIL D'ADMINISTRATION D'UNE QUANTITÉ PRÉDÉFINIE D'UN COMPOSÉ

(30) Priority: 16.02.2021 IT 202100003521
(43) Date of publication of application: 27.12.2023
(73) Proprietor: Foresti, Ruben, 43125 Parma (IT)
(72) Inventor: FORESTI, Ruben, 43125 Parma (IT); ROSSI, Stefano, 46023 Gonzaga (Mantova) (IT); MACALUSO, Claudio, 43121 Parma (IT); MIRAGOLI, Michele, 26100 Cremona (IT); COLOMBO, Paolo, 43125 Parma (IT); SELLERI, Stefano, 40138 Bologna (IT); CATALUCCI, Daniele, 20090 Segrate (Milano) (IT)
(74) Representative: Dondi, Silvia
(86) International application number: PCT/IB2022/051253
(87) International publication number: WO 2022/175796

(56) References cited:
- WO-A1-2015/021064
- WO-A1-98/41254
- US-A1- 2004 055 596
- US-A1- 2011 000 482

## Description

### Technical field

The present invention relates to a process and an apparatus for dispensing a predefined amount of a compound.

The invention has application in the administration of volumes of substances (e.g. drugs) in microlitres to small animals, in particular laboratory animals. For example, the invention is used in preclinical and clinical research.

However, the invention is also applicable in a clinical and therapeutic setting, both on human and animal patients.

The administration by inhalation of substances in gas, liquid or powder form is having exponential growth in preclinical and clinical research, thanks to the enormous development of nano materials for nanomedicine and nanotoxicology studies.

Administration by inhalation is also becoming a sought-after alternative for particular drugs that are less effective and/or reach their target with greater difficulty or more slowly if administered intravenously.

### Background art

Various types of metred-dose inhalers, i.e. pressurised devices used to administer a pharmaceutical product in the respiratory tract by inhalation, are already known.

Such metred-dose inhalers, indicated in the sector by the acronym MDI, comprise a reservoir containing a pharmaceutical formulation in suspension or in solution in a liquid propellent and a metering valve inserted in the reservoir. Thanks to manual actuation of the metering valve it is possible to dispense a dose of a product by means of a propellent that acts as a carrier of the pharmaceutical formulation.

However, in all cases in which it is necessary, in preclinical and clinical research, to administer compounds and/or drugs to small animals or in minimal quantities, the metred-dose inhalers known to date are imprecise and not capable of controlling the volume expelled by inhalation on a level of microlitres. In fact, such inhalers always require the manual intervention of an operator for administration and dispense doses comprised between 20 ul and 100 ul, not being able to reduce the volume below 20 ul.

A metred-dose inhaler wherein an electronic control actuates the opening of a solenoid valve for a pre-established time interval, to which a pre-established volume (dose) of dispensed product corresponds, is known from document WO 2013/083530.

This electronically controlled inhaler is capable of dispensing small volumes of substances, for example from 50 ul to a minimum of 1-2 ul per pulse.

By actuating the valve a number of times though consecutive pulses it is thus possible to reach the total amount to be delivered for a certain treatment.

Moreover, the proposed solution allows two different formulations (placed in two containers) to be dispensed (through two different conduits) in small doses by means of alternating or separate groups of pulses or simultaneous pulses for each formulation.

From documents US 2004/055596 A1 it is known a drug dispenser where the drug is contained inside a reservoir together with the propellent.

From document WO 2015/021064 A1 it is known a dingle dose reusable insufflation device which can be used to administer a compound to small animals by means of a syringe pump.

### Disclosure of the invention

In this context, the technical task at the basis of the present invention is to propose a process and an apparatus for administering a predefined amount of a compound which overcome the above-mentioned drawbacks of the prior art. The invention is defined by the appended claims.

In particular, it is an object of the present invention to propose a process and an apparatus for dispensing a predefined amount of a compound, wherein the compound is delivered in a safer and more precise manner compared to the known solutions.

Another object of the present invention is to provide an apparatus for dispensing a predefined amount of a compound, whose actuation does not require any particular abilities on the part of an operator.

The stated technical task and the specified objects are substantially achieved by a process for administering a predefined amount of a compound to a target, comprising the following steps:
- providing a dosing chamber having a predefined volume;
- disposing the predefined amount of compound downstream of an outlet of the dosing chamber and upstream of the target;
- introducing a propellent into the dosing chamber;
- discharging the propellent from the outlet of the dosing chamber towards the predefined amount of compound so as to dispense said predefined amount of compound to the target.

In accordance with one aspect of the invention, the propellent is a compressed gaseous mixture that is injected into the dosing chamber so that the predefined volume of the dosing chamber is filled by the propellent.

In accordance with one aspect of the invention, the process further comprises a step of filtering the gaseous mixture.

In accordance with one aspect of the invention, the step of discharging the propellent from the outlet of the dosing chamber takes place in a pulsed mode.

In accordance with one aspect of the invention, the process further comprises a step of regulating the pressure of the propellent discharged towards the predefined amount of compound by means of a negative feedback control.

The stated technical task and the specified objects are substantially achieved by an apparatus for administering a predefined amount of a compound to a target, comprising:
- a reservoir containing a gaseous mixture;
- a compressing means for compressing the gaseous mixture contained in the reservoir;
- a dosing chamber having a predefined volume, the dosing chamber having an inlet for the compressed gaseous mixture and an outlet for the compressed gaseous mixture;
- a control unit configured to control the actuation of the compressing means and the delivery of the compressed gaseous mixture from the outlet towards the predefined amount of the compound.

In accordance with one aspect of the invention, the apparatus further comprises a syringe having a cylindrical body constituting the reservoir and a plunger representing the compressing means.

In accordance with one aspect of the invention, the apparatus further comprises a piezoelectric sensor disposed in proximity to the outlet of the dosing chamber and configured to detect a physical parameter representative of the pressure of the compressed mixture.

The control unit is configured to force the pursuit of a nominal pressure for the gaseous mixture in response to the value detected by the piezoelectric sensor.

In accordance with one aspect of the invention, the apparatus further comprises a filtering means upstream or downstream of the dosing chamber.

In accordance with one aspect of the invention, the apparatus comprises further dosing chambers arranged in series downstream of the dosing chamber.

### Brief description of drawings

Additional features and advantages of the present invention will become more apparent from the approximate and thus non-limiting description of a preferred but not exclusive embodiment of a process and an apparatus for dispensing a predefined amount of a compound, as illustrated in the accompanying drawings, in which:

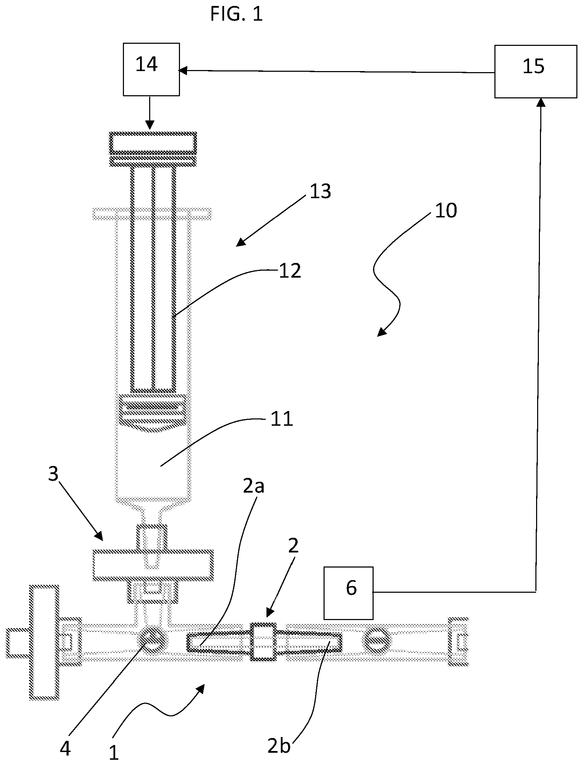

- figure 1 illustrates an apparatus for dispensing a predefined amount of a compound, in accordance with one embodiment of the invention;
- figure 2 illustrates the apparatus in figure 1, in accordance with another embodiment;
- figures 3a and 3b illustrate other embodiments of the device used in the apparatus according to the present invention.

### Detailed description of preferred embodiments of the invention

With reference to the figures, the number 1 indicates a device that may be used to administer a predefined amount of a compound onto a target.

For example, the compound may take on different states: liquid, gas, powder or hydrogel.

In accordance with one aspect of the invention, the compound is a drug and the target is a part of an animal or human body.

The device 1 comprises a dosing chamber 2 having a predefined volume. In this context, the dosing chamber is also called a "lung".

The dosing chamber 2 has at least one inlet 2a for a propellent and an outlet 2b for the discharge of the propellent.

In accordance with one embodiment, the propellent is a compressed gaseous mixture, for example compressed air.

In accordance with a preferred embodiment, the device 1 further comprises a filtering means 3 upstream of the dosing chamber 2.

In accordance with an alternative embodiment (not illustrated), the filtering means 3 is disposed downstream of the dosing chamber 2.

The number 10 indicates an apparatus comprising:
- the device 1;
- a reservoir 11 containing the gaseous mixture (for example air)
- a compressing means 12 for compressing the gaseous mixture contained in the reservoir 11.

In the embodiment described and illustrated herein, the system 10 comprises a syringe 13 having a hollow cylindrical body that performs the action of a reservoir 11 for the gaseous mixture and a plunger that constitutes the compressing means 12 for compressing the gaseous mixture.

The device 1 comprises a valve 4 configurable at least in:
- a first position in which it enables fluid communication between the reservoir 11 (e.g. the cylindrical body of the syringe 13) and the dosing chamber 2;
- a second position in which it interrupts the fluid communication between the reservoir 11 and the dosing chamber 2.

For example, in the embodiment described and illustrated herein, the valve 4 is interposed between the hollow cylindrical body 11 of the syringe 13 and the inlet 2a of the dosing chamber 2.

The fluid communication between the hollow cylindrical body 11 of the syringe 13 and the inlet 2a of the dosing chamber 2 can take place through one or more tubes and/or connectors, inside which the valve 4 is inserted.

In accordance with the proposed process, the predefined amount of compound to be administered to the target is disposed downstream of the outlet 2b of the dosing chamber 2.

As will be seen further below, the provision of said predefined amount can take place in different ways.

For the purpose of administering said predefined amount of compound to the target, in accordance with one embodiment, the process comprises the following steps:
- compressing the gaseous mixture;
- introducing the compressed gaseous mixture into the dosing chamber 2 through the inlet 2a so as to fill the predefined volume of the dosing chamber 2;
- discharging the compressed gaseous mixture from the outlet 2b of the dosing chamber 2 so as to make it act like a propellent on the predefined amount of compound disposed downstream of the outlet 2b itself.

In accordance with the embodiment described and illustrated herein, the gaseous mixture contained in the cylindrical body 11 of the syringe 13 is compressed by pushing the plunger 12.

The actuation of the plunger 12 may take place manually or else by means of an automatic actuation means.

In the latter case, the device 1 also comprises the automatic actuation means 14, which is configured to push the plunger 12 and thereby load the propellent into the dosing chamber 2 through the inlet 2a.

The automatic actuation means 14 preferably comprises a linear motor controlled by a control unit 15.

In accordance with one embodiment, the control unit 15 is configured for a feedback control of the pressure of the propellent.

For example, the control unit 15 receives a signal from a piezoelectric sensor 6 disposed in proximity to the outlet 2b of the dosing chamber 2 and configured to detect a physical parameter representative of the pressure of the propellent in that zone (i.e. at the outlet 2b).

In particular, the piezoelectric sensor 6 detects a force value of the propellent.

In response to the force value detected by the piezoelectric sensor 6, the control unit 15 is configured to calculate the corresponding pressure and force the pursuit of a nominal pressure value.

The nominal pressure is selected so that the delivery of the predefined volume of propellent is not damaging for the target. Therefore, the control unit 15 renders the administration of the compound safer. This is fundamental for certain types of application; one need only think of use in a veterinary setting on laboratory guinea pigs.

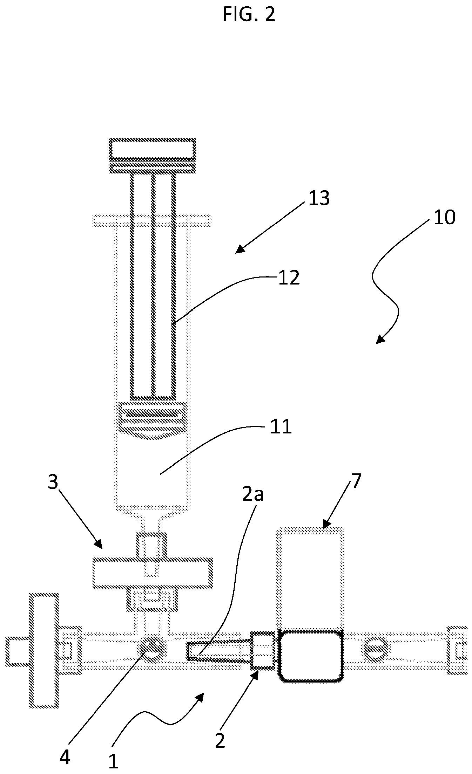

In accordance with one embodiment, illustrated in figure 2, the device 1 also comprises a solenoid valve 7 located downstream of the dosing chamber 2 and configured to discharge the propellent towards the predefined amount of compound in a pulse mode.

The device 1 described thus far is a single-stage device in that it comprises a single dosing chamber 2, i.e. a single "lung".

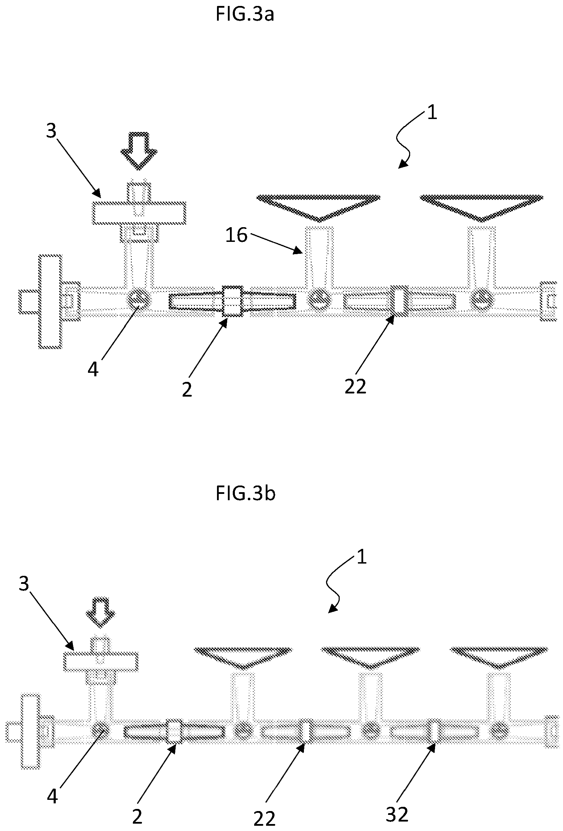

In alternative embodiments, illustrated in figures 3a and 3b, the device 1 comprises further dosing chambers 22, 32 (or lungs) arranged in series; therefore, it is a multi-stage device.

Different configurations can be created, with several lungs, with several inlets for different substances that will make up the dose of the compound to be delivered (e.g. powder entering from one stage, liquid entering from another stage, etc.), with or without a solenoid valve for pulsed administration, etc.

For example, figure 3a illustrates a second lung 22 that receives the propellent from the first lung 2 and the dose of the compound to be administered to the target from an intermediate conduit 16 between the first lung 2 and the second lung.

In accordance with another embodiment, the process comprises a step of aspirating the gaseous mixture into the dosing chamber 2 through a means for creating a vacuum (means of a known type).

In accordance with one embodiment, the compound is in the form of a tablet from which a predefined amount in powder form is derived.

The device 1 preferably also comprises a site for housing the tablet.

The features of a process and an apparatus for administering a predefined amount of a compound according to the present invention are clear from the description, as are the advantages.

The proposed process and apparatus allow the predefined amount of the compound to be administered safely to the patient (human or animal), without requiring any particular abilities on the operator's part.

In fact, the compound is dosed by means of a predefined volumetric amount of propellent (air or another gaseous mixture under pressure), thereby ensuring that the pressure is kept under control and preventing peaks from being reached which could harm the target.

Furthermore, the propellent acts as a carrier of the compound directly in proximity of the target, i.e. it hits the dose of compound exiting from the dosing chamber, thus preventing residues of the compound from accumulating upstream and not being dispensed onto the target. In the known solutions, by contrast, the compound pre-mixed with the propellent is dispensed from a metering valve and residues of the compound can thus accumulate in the latter. In such solutions, not all of the dose of the compound is dispensed onto the target.

Furthermore, the proposed solution is extremely versatile, since lungs of different volumes can be provided to dose different amounts of a compound.

Furthermore, certain pharmaceutical compounds must be constituted shortly before administration. In such a case, the device used in the proposed apparatus makes it possible to provide different inlets for the components (powder, liquid, etc.) to be mixed in order to constitute the desired compound.

The presence of a control unit, moreover, enables administration to be automated, rendering delivery completely independent of the operator's manual ability.

Precisely the automation allows the delivery of small volumes of a compound, ranging from 0.037 I/min to 0.174 I/min, and which would be impossible to control by means of the prior art devices manually actuated by the operator.

Moreover, the feedback control of pressure enables the level of safety of the apparatus to be further increased.

Furthermore, with the process and the apparatus proposed herein one obtains a higher precision of delivery than that in document WO 2013/083530, in which the control of the volume was obtained indirectly by monitoring the time of administration, i.e. the opening interval of the solenoid valve.

## Claims

1. An apparatus (10) for administering a predefined amount of a compound to a target, comprising:
- a reservoir (11) containing a gaseous mixture;
- a compressing means (12) for compressing the gaseous mixture contained in the reservoir (11);
- a dosing chamber (2) having a predefined volume, said dosing chamber (2) having an inlet (2a) for the compressed gaseous mixture and an outlet (2b) for said compressed gaseous mixture, said predefined amount of compound being disposed downstream of the outlet (2b) of the dosing chamber (2), said compressed gaseous mixture dicharged from the outlet (2b) of the dosing chamber (2) acting as a propellent on the predefined amount of compound;
- a control unit (15) configured to control the actuation of the compressing means (12) and the delivery of the compressed gaseous mixture from the outlet (2b) towards the predefined amount of the compound.

2. The apparatus (10) according to claim 1, further comprising a syringe (13) having a cylindrical body constituting said reservoir (11) and a plunger (12) constituting said compressing means (12).

3. The apparatus (10) according to claim 1 or 2, further comprising a piezoelectric sensor (6) disposed in proximity to the outlet (2b) of the dosing chamber (2) and configured to detect a physical parameter representative of the pressure of the compressed mixture, said control unit (15) being configured to force the pursuit of a nominal pressure for the gaseous mixture in response to the value detected by the piezoelectric sensor (6).

4. The apparatus (10) according to any one of claims 1 to 3, further comprising a filtering means (3) upstream or downstream of the dosing chamber (2).

5. The apparatus (10) according to any one of claims 1 to 4, comprising further dosing chambers (22, 32) arranged in series downstream of said dosing chamber (2).

## Patentansprüche

1. Vorrichtung (10) zur Verabreichung einer vorbestimmten Menge einer Verbindung an ein Ziel, umfassend:
- ein Reservoir (11), das ein Gasgemisch enthält;
- Verdichtungsmittel (12) zum Verdichten des in dem Reservoir (11) enthaltenen Gasgemisches;
- eine Dosierkammer (2) mit einem vorbestimmten Volumen, wobei die Dosierkammer (2) einen Einlass (2a) für das verdichtete Gasgemisch und einen Auslass (2b) für das verdichtete Gasgemisch aufweist, wobei die vorbestimmte Menge an Verbindung stromabwärts des Auslasses (2b) der Dosierkammer (2) angeordnet ist, wobei das verdichtete Gasgemisch, das aus dem Auslass (2b) der Dosierkammer (2) entladen wird, als Treibmittel auf die vorbestimmte Menge an Verbindung wirkt;
- eine Steuereinheit (15), die ausgelegt ist, um die Betätigung der Verdichtungsmittel (12) und die Abgabe des verdichteten Gasgemisches vom Auslass (2b) in Richtung der vorbestimmten Menge der Verbindung zu steuern.

2. Vorrichtung (10) nach Anspruch 1, ferner umfassend eine Spritze (13) mit einem zylindrischen Körper, der das Reservoir (11) bildet, und einem Kolben (12), der die Verdichtungsmittel (12) bildet.

3. Vorrichtung (10) nach Anspruch 1 oder 2, ferner umfassend einen piezoelektrischen Sensor (6), der in der Nähe des Auslasses (2b) der Dosierkammer (2) angeordnet und ausgelegt ist, um einen physikalischen Parameter zu erfassen, der für den Druck des verdichteten Gemisches repräsentativ ist, wobei die Steuereinheit (15) ausgelegt ist, um die Verfolgung eines Nenndrucks für das Gasgemisch als Reaktion auf den durch den piezoelektrischen Sensor (6) erfassten Wert zu erzwingen.

4. Vorrichtung (10) nach einem der Ansprüche 1 bis 3, ferner umfassend Filtermittel (3) stromaufwärts oder stromabwärts der Dosierkammer (2).

5. Vorrichtung (10) nach einem der Ansprüche 1 bis 4, umfassend weitere Dosierkammern (22, 32), die in Reihe stromabwärts der Dosierkammer (2) angeordnet sind.

## Revendications

1. Appareil (10) d'administration d'une quantité prédéfinie d'un composé à une cible, comprenant :
- un réservoir (11) contenant un mélange gazeux ;
- des moyens de compression (12) servant à comprimer le mélange gazeux contenu dans le réservoir (11) ;
- une chambre de dosage (2) ayant un volume prédéfini, ladite chambre de dosage (2) comportant une entrée (2a) pour le mélange gazeux comprimé et une sortie (2b) pour ledit mélange gazeux comprimé, ladite quantité prédéfinie de composé étant disposée en aval de la sortie (2b) de la chambre de dosage (2), ledit mélange gazeux comprimé déchargé de la sortie (2b) de la chambre de dosage (2) agissant comme un propulseur sur la quantité prédéfinie de composé ;
- une unité de contrôle (15) configurée pour contrôler l'actionnement des moyens de compression (12) et la distribution du mélange gazeux comprimé de la sortie (2b) vers la quantité prédéfinie du composé.

2. Appareil (10) selon la revendication 1, comprenant en outre une seringue (13) ayant un corps cylindrique constituant ledit réservoir (11) et un piston (12) constituant lesdits moyens de compression (12).

3. Appareil (10) selon la revendication 1 ou 2, comprenant en outre un capteur piézoélectrique (6) disposé à proximité de la sortie (2b) de la chambre de dosage (2) et configuré pour détecter un paramètre physique représentatif de la pression du mélange comprimé, ladite unité de contrôle (15) étant configurée pour forcer la poursuite d'une pression nominale pour le mélange gazeux en réponse à la valeur détectée par le capteur piézoélectrique (6).

4. Appareil (10) selon l'une quelconque des revendications 1 à 3, comprenant en outre des moyens de filtrage (3) en amont ou en aval de la chambre de dosage (2).

5. Appareil (10) selon l'une quelconque des revendications 1 à 4, comprenant des outres chambres de dosage (22, 32) disposées en série en aval de ladite chambre de dosage (2).
